# EUROPÄISCHE PATENTANMELDUNG

(11) **EP 1 880 690 A1**
(43) Veröffentlichungstag der Anmeldung: **23.01.2008**
(21) Anmeldenummer: 07013240.2
(22) Anmeldetag: 06.07.2007
(51) Int. Cl.: A61C 8/00

(54) **Dentales Implantatsystemteil mit einer Beschichtung**

(30) Priorität: 17.07.2006 DE 102006033312
(71) Anmelder: Heraeus Kulzer GmbH, 63450 Hanau (DE)
(72) Erfinder: Kühn, Klaus-Dieter, Dr., 35041 Marburg (DE); Martinovic, Sandra, Dr., 60594 Frankfurt (DE); Vogt, Sebastian, Dr., 99092 Erfurt (DE)
(74) Vertreter: Kühn, Hans-Christian

(57) **Zusammenfassung**

Die Erfindung betrifft ein dentales Implantatsystemteil mit einer Beschichtung aus einem in Wasser gering löslichen Chlorhexidinfettsäuresalz, in der ein oder mehrere Antiphlogistika und/oder ein oder mehrere Steroide und/oder ein oder mehrere Prostaglandine und/oder ein oder mehrere Bisphosphonate und/oder ein oder mehrere Statine gelöst sind.

## Beschreibung

Unter dem Begriff dentales Implantatsystemteil werden Einheilkappen, Gingivaformer, Verbindungselemente, Implantatkörper und Implantataufbauten verstanden.

Teile eines Implantatsystems können grundsätzlich in 2 Gruppen unterteilt werden: in die dauerhaft im Knochen verbleibenden, nicht-austauschbaren Teile (z.B. der Implantatkörper) und die austauschbaren Teile, die je nach Behandlungsphase eingesetzt und ausgetauscht werden können (z.B. die Gingivaformer).

In der dentalen lmplantologie wird nach der Implantation des Implantatkörpers in den Kieferknochen der Implantatkörper durch eine Einheilkappe während der Einheilphase verschlossen. Alternativ kann ein Implantat auch transgingival oder offen einheilen. Nach erfolgter Einheilphase, die bis zu sechs Monaten dauern kann, wird zur Ausheilung und Ausformung des Weichgewebes die Einheilkappe entfernt und durch einen Gingivaformer ersetzt. Nach erfolgter Ausheilung und Ausformung des Weichgewebes erfolgt die Entfernung des Gingivaformers und das Einsetzen eines definitiven lmplantataufbaus auf den Implantatkörper. Synonym für den Implantataufbau sind die Begriffe Abutment, Aufbau-Pfosten, Implantat-Pfosten und Insert.

Nach der Implantation des Implantatkörpers kann es aus der Mundhöhle zu einer mikrobiellen Besiedlung der Oberflächen des Implantatkörpers und der Einheilkappe kommen sowie später auch des Gingivaformers.

In der frühen Phase kann diese mikrobielle Besiedelung zu einer Entzündung rund um den Implantatkörper führen, die bei geringer Ausprägung zu begrenztem Knochenverlust im krestalen Bereich führen kann und bei starker Ausprägung zu einem kompletten Implantatverlust. Der Knochenverlust im krestalen Bereich ist die häufigere Komplikation.

Langfristig kann die Besiedelung der Implantatoberflächen zu einer periimplantären Mukositis führen, die wiederum einen Knochenverlust und im Extremfall einen Implantatverlust nach sich ziehen kann.

In der Frühphase handelt es sich um eine akute Entzündungsreaktion, während später eher chronische Mechanismen eine Rolle spielen.

Um diesen Prozessen vorzubeugen, ist es wünschenswert, dentale Implantatsystemteile zu verwenden, die an ihrer Oberfläche gegenüber einer mikrobiellen Besiedlung geschützt sind. Weiterhin ist es wünschenswert, dass entzündliche Prozesse in der näheren Umgebung von dentalen Implantatsystemteilen während der frühen Einheilphase vermieden werden.

Der Erfindung liegt die Aufgabe zu Grunde, eine dentales lmplantatsystemteil bereitzustellen, das die Gewebeheilung beim Patienten verbessert und möglichst Entzündungen vermeidet.

Die Aufgabe wird erfindungsgemäß durch die Merkmale des Anspruchs 1 gelöst, Bevorzugte Ausführungsformen sind in den Unteransprüchen angegeben. Es wurde ein dentales Implantatsystemteil entwickelt, bei dem das dentale Implantatsystemteil mit einer Schicht mit mindestens einem in Wasser gering löslichen Chlorhexidinfettsäuresalz beschichtet ist, in dem ein oder mehrere Antiphlogistika und/oder ein oder mehrere Steroide und/oder ein oder mehrere Prostaglandine und/oder ein oder mehrere Bisphosphonate und/oder ein oder mehrere Statine gelöst sind, wobei die Schicht in Wasser bevorzugt basisch oder pH-neutral reagiert. Der Erfindung liegt der überraschende Befund zu Grunde, dass Chlorhexdinfettsäuresalze schichtbildend sind und dass sich in Chlorhexidinfettsäuresalzen unter anderem nichtsteroidale und auch steroidale Antiphlogistika lösen. Der besondere Vorteil des erfindungsgemäßen Implantatsystemteils liegt darin, dass die Geometrie und die stoffliche Zusammensetzung der Einheilkappen und Gingiva-Former variabel sein können, weil die Schicht auf unterschiedlichsten Oberflächen haftet. Durch die Erfindung wird ein Implantatteil zur Verfügung gestellt, das gegenüber einer mikrobiellen Besiedlung über einen Zeitraum von mehreren Tagen bis zu einigen Wochen geschützt ist. Das dentale lmplantatsystemteil soll bevorzugt nur an der Oberfläche antiseptisch ausgerüstet sein.

Es ist vorteilhaft, dass Chlorhexidinlaurat, Chlorhexidinmyristat, Chlorhexidinpalmitat und/oder Chlorhexidinstearat als Chlorhexidinfettsäuresalze verwendet werden. Diese Chlorhexidinfettsäuresalze sind in Wasser gering löslich und geben Chlorhexidin über einen Zeitraum von mehreren Tagen bis Wochen im wässrigen Milieu ab. Die geringe Löslichkeit bringt den Vorteil mit sich , dass nur die Oberfläche des Implantatsystemteils antimikrobiell geschützt ist und dass keine größeren Mengen Chlorhexidin in die Umgebung der temporären, dentalen Implantamaterialien abgegeben werden.

Bevorzugt wird Ibuprofen, Indomethacin und/oder Diclofenac als Antiphlogistika verwendet. Es ist auch vorteilhaft, dass die Antiphlogistika in der Säureform vorliegen. Die Säureformen dieser Antiphlogistika lösen sich in Chlorhexidinfettsäureestern. Dagegen sind die Alkali-Salzformen dieser Antiphlogistika in Chlorhexidinfettsäuresalzen nicht löslich. Die Säureformen der Antiphlogistika haben gegenüber den Salzformen den Vorteil, dass sie in Wasser oder wässrigem Milieu nur gering löslich sind. Dadurch ist es möglich, dass die Antiphlogistika retardiert aus den schichtbildenden Chlorhexidinfettsäuresalzen in Gegenwart von Wasser oder wässrigem Milieu herausgelöst werden.

Zweckmäßig ist weiterhin, dass Kortikosteroide und/oder Östrogene als Steroide verwendet werden. Es werden Triamcinolon, Dexamethason und/oder Östradiol als Steroide besonders bevorzugt. Diese Steroide lösen sich ebenfalls in Chlorhexidinfettsäuresalzen und sind im wässrigen Milieu nur gering löslich. Weitere Kortikosteroide, wie Betamethason, Methylprednisolon, Prednison, Prednisolon, Cortison und/oder Hydrocortison können eingesetzt werden.

Erfindungsgemäß ist ebenfalls, dass Prostaglandine in der Schicht enthalten sind. Prostaglandine können in Zusammenwirken mit antiphlogistischen Wirkstoffen synergistisch wirken. Vorteilhafterweise wird Prostaglandin E2 verwendet.

Im Rahmen der Erfindung ist auch, dass Bisphosphonate in der Beschichtung enthalten sind. Die Bisphosphonate liegen dabei bevorzugt in der Säureform vor.
Das Bisphosphonat Aledronat wird besonders bevorzugt.

Vorteilhafterweise werden Simavastatin und/oder Lovastatin in der Beschichtung als Statine verwendet.

Die Erfindung wird durch nachstehende Beispiele erläutert, ohne sie dadurch zu beschränken.

### Beispiel 1:

Es werden die der Schleimhaut zugewandten Oberflächen einer auf 70 °C erwärmten Einheilkappe, die eine Masse von 793,6 mg hat und aus Titan gefertigt ist, mit einer 5 %igen ethanolischen Lösung von Chlorhexidinpalmitat, in der 1 % Ibuprofen gelöst ist, besprüht. Es bildet sich eine transparente Schicht. Die Masse der Beschichtung beträgt 2,1 mg.

### Beispiel 2:

Es werden die der Schleimhaut zugewandten Oberflächen einer auf 70 °C erwärmten Einheilkappe, die eine Masse von 766,9 mg hat und aus Titan gefertigt ist, mit einer 5 %igen ethanolischen Lösung von Chlorhexidinpalmitat, in der 1 % Diclofenac gelöst ist, besprüht. Es bildet sich eine transparente Schicht. Die Masse der Beschichtung beträgt 1,7 mg.

### Beispiel 3:

Es werden die der Schleimhaut zugewandten Oberflächen einer auf 70°C erwärmten Einheilkappe, die eine Masse von 753,9 mg hat und aus Titan gefertigt ist, mit einer 5%igen ethanolischen Lösung von Chlorhexidinpalmitat, in der 1 % Prostaglandin E2, welches selektiv auf EP4 Rezeptoren wirkt, gelöst ist, besprüht. Es bildet sich eine transparente Schicht.

### Beispiel 4:

Es werden die der Schleimhaut zugewandten Oberflächen einer auf 70°C erwärmten Einheilkappe, die eine Masse von 791,39 mg hat und aus Titan gefertigt ist, mit einer 5%igen ethanolischen Lösung von Chlorhexidinpalmitat, in der 1 % Simvastatin, gelöst ist, besprüht. Es bildet sich eine transparente Schicht.

### Beispiel 5:

Es werden die der Schleimhaut zugewandten Oberflächen einer auf 70°C erwärmten Einheilkappe, die eine Masse von 791,39 mg hat und aus Titan gefertigt ist, mit einer 5%igen ethanolischen Lösung von Chlorhexidinpalmitat, in der 1% Aledronat, gelöst ist, besprüht. Es bildet sich eine transparente Schicht.

### Beispiel 6:

Es werden die der Schleimhaut zugewandten Oberflächen einer auf 70°C erwärmten Einheilkappe, die eine Masse von 791,39 mg hat und aus Titan gefertigt ist, mit einer 5%igen ethanolischen Lösung von Chlorhexidinpalmitat, in der 1 % Dexamethason, gelöst ist, besprüht. Es bildet sich eine transparente Schicht.

### Beispiel 7:

Es werden die der Schleimhaut zugewandten Oberflächen einer auf 70°C erwärmten Einheilkappe, die eine Masse von 791,39 mg hat und aus Titan gefertigt ist, mit einer 5%igen ethanolischen Lösung von Chlorhexidinpalmitat, in der 1% Estradiol, gelöst ist, besprüht. Es bildet sich eine transparente Schicht.

## Patentansprüche

1. Dentales Implantatsystemteil mit einer Beschichtung aus einem in Wasser gering löslichen Chlorhexidinfettsäuresalz, in der ein oder mehrere Antiphlogistika und/oder ein oder mehrere Steroide und/oder ein oder mehrere Prostaglandine und/oder ein oder mehrere Bisphosphonate und/oder ein oder mehrere Statine gelöst sind.

2. Dentales Implantatsystemteil nach Anspruch 1, **dadurch gekennzeichnet, dass** als Chlorhexidinfettsäuresalz(e) Chlorhexidinlaurat, Chlorhexidinmyristat, Chlorhexidinpalmitat und/oder Chlorhexidinstearat verwendet wird/werden.

3. Dentales Implantatsystemteil nach Anspruch 1 oder 2, **dadurch gekennzeichnet, dass** die Beschichtung in Wasser basisch oder pH-neutral reagiert.

4. Dentales Implantatsystemteil nach einem der Ansprüche 1 bis 3, **dadurch gekennzeichnet, dass** Ibuprofen, Indomethacin und/oder Diclofenac als Antiphlogistika verwendet werden.

5. Dentales Implantatsystemteil nach einem der Ansprüche 1 bis 4, **dadurch gekennzeichnet, dass** die Antiphlogistika in der Säureform vorliegen.

6. Dentales lmplantatsystemteil nach einem der Ansprüche 1 bis 5, **dadurch gekennzeichnet, dass** Kortikosteroide und Östrogene als Steroide verwendet werden.

7. Dentales Implantatsystemteil nach einem der Ansprüche 1 bis 6, **dadurch gekennzeichnet, dass** Triamcinolon, Dexamethason und/oder Östradiol als Steroide verwendet werden.

8. Dentales Implantatsystemteil nach einem der Ansprüche 1 bis 7, **dadurch gekennzeichnet, dass** Prostaglandin E2 verwendet wird.

9. Dentales Implantatsystemteil nach einem der Ansprüche 1 bis 8, **dadurch gekennzeichnet, dass** als Bisphosphonat Aledronat verwendet wird.

10. Dentales lmplantatsystemteil nach einem der Ansprüche 1 bis 9, **dadurch gekennzeichnet, dass** das Bisphosphonat in der Säureform vorliegt.

11. Dentales Implantatsystemteil nach einem der Ansprüche 1 bis 10, **dadurch gekennzeichnet, dass** Lovastatin und/oder Simavastatin als Statine verwendet werden.
